# EUROPEAN PATENT APPLICATION

(11) **EP 4 483 980 A1**
(43) Date of publication of application: **01.01.2025**
(21) Application number: 22924354.8
(22) Date of filing: 29.11.2022
(51) Int. Cl.: B01D 11/04, B01D 1/30, B01D 35/02, A61K 9/16

(54) **SOLVENT REMOVING APPARATUS AND METHOD OF MANUFACTURING MICROSPHERE USING SAME**

(30) Priority: 26.01.2022 KR 20220011526
(71) Applicant: Inventage Lab. Inc., Seongnam-si, Gyeonggi-do 13438 (KR)
(72) Inventor: KIM, Dong Hoon, Seongnam-si Gyeonggi-do 13530 (KR); CHON, Chan Hee, Seongnam-si Gyeonggi-do 13165 (KR); KIM, Ju Hee, Seongnam-si Gyeonggi-do 13438 (KR)
(74) Representative: BCKIP Part mbB
(86) International application number: PCT/KR2022/019031
(87) International publication number: WO 2023/146101

(57) **Abstract**

A solvent removing apparatus includes a container accommodating an emulsion including a first raw material in a continuous phase and a second raw material in a dispersed phase, a filter unit connected to the inside of the container, receiving the emulsion from the container, filtering a portion of the continuous phase of the emulsion and the solvent in the continuous phase, and then supplying the remaining emulsion back to the container, a supply unit connected to the inside of the container and supplying the first raw material to the inside of the container, and a stirring device for stirring the emulsion in the container by generating a flow.

## Description

### FIELD OF THE INVENTION

The present disclosure relates to a solvent removing apparatus and a method of preparing a microsphere using the solvent removing apparatus, and more specifically, to a solvent removing apparatus for extracting and removing a solvent of an emulsion used in preparing a microsphere, and a method of preparing a microsphere using the solvent removing apparatus.

### BACKGROUND ART

One of drug delivery systems currently being actively researched and developed, and used is a so-called polymeric drug delivery system ("PDDS"), which enables a controlled release of a certain amount of therapeutic agent over a long period in a circulating dosage for both hydrophilic and hydrophobic therapeutic agents using a biodegradable, biocompatible, and nontoxic polymer, such as a polylactic acid (PLA)/polyglycolic (PGA) polymer.

Such a biodegradable polymer may be prepared in the form of a microsphere by various known techniques. When these biodegradable polymer microspheres are prepared, the most frequently used method is to dissolve the biodegradable polymer or the substance to be encapsulated (drug or other active agent) with the biodegradable polymer in a solvent using a known method and disperse or emulsify the same in an aqueous solution containing a surfactant. Subsequently, the solvent is dried after being removed from the microspheres to obtain a microsphere product. Since toxic solvents such as dichloromethane or chloroform are mainly used to dissolve the biodegradable polymer and active medication in the process of preparing a microsphere using known technologies, sufficient time and effort need to be spent on removing the solvent so that these solvents do not remain in the final microsphere product, which increases the time required to obtain the microsphere product and acts as an obstacle to mass production. Therefore, efforts have been made to mass-produce high-quality microspheres at low cost.

In particular, technologies related to various solvent removal apparatuses for removing solvents are being developed, but generally, the solvent is extracted and removed through stirring of the emulsion using an impeller or stirrer coupled to a rotational shaft that rotates using a motor, but a more effective method for this has not been presented.

### PROBLEM TO BE SOLVED

The present disclosure is directed to providing a solvent removing apparatus for more efficiently extracting and removing a solvent of an emulsion used to prepare a microsphere.

The present disclosure is also directed to providing a method of preparing a microsphere using the solvent removing apparatus.

### TECHNICAL MEANS TO SOLVE THE PROBLEM

To achieve the above objects of the present disclosure, a solvent removing apparatus according to one embodiment includes a container that accommodates an emulsion including a first raw material in a continuous phase and a second raw material in a dispersed phase, a filter unit connected to the inside of the container to receive the emulsion from the container and filter a solvent in the continuous phase together with a portion of the continuous phase of the emulsion, a supply unit connected to the inside of the container to supply the first raw material to the inside of the container, and a stirring device that stirs the emulsion in the container by generating a flow.

In one embodiment of the present disclosure, the filter unit may include a tangential flow filter (TFF) formed to filter the portion of the continuous phase of the emulsion and the solvent in the continuous phase and then provide the remaining emulsion back to the container. The TFF may filter a solvent and a portion of the first raw material without filtering a dispersed phase of the emulsion.

In one embodiment of the present disclosure, the TFF may be formed to filter particles of 10 µm (micrometers) or less.

In one embodiment of the present disclosure, the solvent removing apparatus may further include an evaporation condensation unit that evaporates the filtered solvent and portion of the first material and condenses only the first raw material. The first raw material recovered from the evaporation condensation unit may be provided to the supply unit to supply the recovered first raw material back to the container.

In one embodiment of the present disclosure, a first raw material equivalent to the amount of the first raw material filtered by the filter unit or the amount of the solvent and first raw material filtered by the filter unit may be supplied from the supply unit to the container. A constant fluid level in the container may be maintained.

In one embodiment of the present disclosure, the solvent removing apparatus may further include a fluid level sensor that measures a fluid level in the container. The fluid level in the container may be constantly controlled by adjusting the amount of the first raw material supplied from the supply unit using the measured fluid level.

In one embodiment of the present disclosure, a portion of the filter unit connected to the container may be formed at the top of the container. The supply unit may provide the first raw material to the bottom of the container.

In one embodiment of the present disclosure, the solvent removing apparatus may further include a heating device that heats the emulsion in the container.

In one embodiment of the present disclosure, a feeding speed of the emulsion provided from the container to the filter unit may be faster than a feeding speed of the first raw material provided from the supply unit so that the fluid level in the container is maintained at the portion of the filter unit connected to the inside of the container.

In one embodiment of the present disclosure, a temperature of the first raw material supplied from the supply unit may be lower than a temperature of the emulsion provided from the container to the filter unit.

In one embodiment of the present disclosure, the first raw material may include purified water and a surfactant, and the second raw material may include an organic solvent, a biodegradable polymer, and a drug.

To achieve the above objects of the present disclosure, a method of preparing a microsphere may use a solvent removing apparatus including a container, a filter unit connected to an inside of the container, a supply unit connected to the inside of the container, and a stirring device disposed in the container. The method of preparing a microsphere includes an operation of preparing a first raw material and a second raw material including a biodegradable polymer, a drug, and a solvent, an operation of forming an emulsion including the first raw material in a continuous phase and the second raw material in a dispersed phase using the first raw material and the second raw material, an operation of providing the emulsion to the inside of the container of the solvent removing apparatus, an operation of stirring the emulsion using the stirring device in the solvent removing apparatus and extracting the solvent in the dispersed phase of the emulsion as the continuous phase, an operation of filtering a portion of the continuous phase including the extracted solvent and the solvent in the continuous phase using the filter unit and then providing the remaining emulsion back to the container, and an operation of supplying a solution in a continuous phase to the container.

In one embodiment of the present disclosure, in the operation of the filtering, the filter unit may include a tangential flow filter (TFF), and the TFF may filter particles of 10 µm (micrometers) or less in order to filter a solvent and a portion of the first raw material without filtering the dispersed phase of the emulsion.

In one embodiment of the present disclosure, the extracting operation may include heating the emulsion in the container to accelerate extraction and evaporation of the solvent in the dispersed phase. In the supplying operation, a temperature of the first raw material supplied from the supply unit may be lower than a temperature of the emulsion provided from the container to the filter unit.

In one embodiment of the present disclosure, the method may further include an evaporating and condensing operation of evaporating the solvent and portion of the first material filtered by the filter unit and condensing only the first raw material. The supplying operation may include supplying a first raw material recovered from the evaporation condensation unit to the container.

In one embodiment of the present disclosure, in the operation of the filtering, the filter unit may receive the emulsion at the top of the container and filter a portion of the continuous phase and the solvent in the continuous phase. In the supplying operation, the solution in the continuous phase may be provided to the bottom of the container.

In one embodiment of the present disclosure, in the preparing operation, the first raw material includes purified water and a surfactant, and the second raw material may include an organic solvent, a biodegradable polymer, and a drug.

### EFFECT OF THE INVENTION

According to the embodiments of the present disclosure, a solvent removing apparatus includes a container accommodating an emulsion including a first raw material in a continuous phase and a second raw material in a dispersed phase, a filter unit connected to the inside of the container, receiving the emulsion from the container, filtering a portion of the continuous phase of the emulsion and the solvent in the continuous phase, and then supplying the remaining emulsion back to the container, a supply unit connected to the inside of the container and supplying the first raw material to the inside of the container, and a stirring device for stirring the emulsion in the container by generating a flow. Since the solvent in the dispersed phase of the emulsion is extracted and evaporated in the continuous phase, the solvent extracted in the continuous phase is removed by the filter unit, and the first raw material is supplied and circulated by the supply unit, it is possible to efficiently extract and remove the solvent.

However, effects of the present disclosure are not limited to the above effects and may be expanded in various ways without departing from the spirit and scope of the present disclosure.

### BRIEF DESCRIPTION OF THE DRAWINGS

FIG. 1 is a view showing a system for preparing a microsphere including a solvent removing apparatus according to one embodiment of the present disclosure.
FIG. 2 is a view showing the solvent removing apparatus according to one embodiment of the present disclosure.
FIG. 3 is a view showing a filter unit of the solvent removing apparatus of FIG. 2.
FIG. 4 is a view showing a solvent removing apparatus according to another embodiment of the present disclosure.
FIG. 5 is a view showing a solvent removing apparatus according to still another embodiment of the present disclosure.
FIG. 6 is a view showing a solvent removing apparatus according to yet another embodiment of the present disclosure.
FIG. 7 is a flowchart showing a method of preparing a microsphere according to one embodiment of the present disclosure.
FIG. 8 is a flowchart showing a solvent extracting removing operation of the method of preparing a microsphere of FIG. 7.
FIG. 9 is a schematic view for describing a detailed principle of formation of microspheres using a microchip.

### DETAILED DESCRIPTION OF EXEMPLARY EMBODIMENTS

Hereinafter, exemplary embodiments of the present disclosure will be described in more detail with reference to the accompanying drawings.

Since the present invention can be variously modified and may have various forms, specific embodiments will be illustrated in drawings and described in detail in the detailed description. However, it should be understood that this is not intended to limit the present invention to a specific disclosed form and includes all changes, equivalents, and substitutions included in the spirit and technical scope of the present invention.

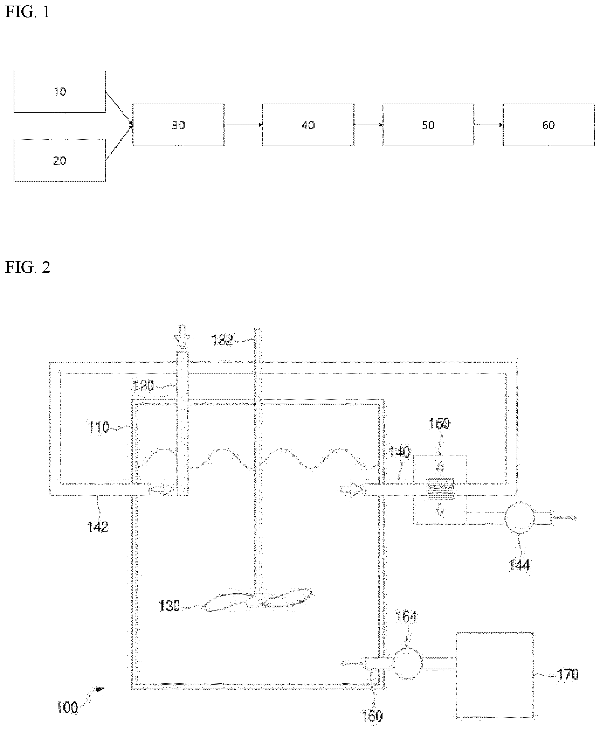

FIG. 1 is a view showing a system for preparing a microsphere including a solvent removing apparatus according to one embodiment of the present disclosure.

Referring to FIG. 1, the system for preparing a microsphere includes a raw material storage unit, an emulsion generation unit 30, a solvent extraction removal unit 40, a cleaning unit 50, and a drying unit 60. The raw material storage unit may include a first raw material storage 10 and a second raw material storage 20.

The first raw material storage 10 may store a first raw material. The first raw material may include purified water and a surfactant. For example, the first raw material may be an aqueous solution in which polyvinyl alcohol ("PVA") is dissolved as a surfactant in purified water.

The type of the surfactant is not particularly limited, and any surfactant that can help form a stable droplet dispersion phase in an aqueous solution in which a biodegradable polymer solution is a continuous phase may be used. The surfactant may preferably be selected from the group consisting of methylcellulose, polyvinylpyrrolidone, carboxymethylcellulose, lecithin, gelatin, polyvinyl alcohol, polyoxyethylene sorbitan fatty acid ester, and polyoxyethylene castor oil derivatives and a mixture thereof.

The second raw material storage 20 may store a second raw material. The second raw material may be an oil-phase solution and may include an organic solvent, a biodegradable polymer dissolved therein, and a drug. The organic solvent may be a solvent used to dissolve the biodegradable polymer and may have a property of not being miscible with water. The type of organic solvent that dissolves the biodegradable polymer is not particularly limited, but preferably, may be one or more selected from the group consisting of dichloromethane, chloroform, ethyl acetate, acetone, acetonitrile, dimethyl sulfoxide, dimethyl formamide, methyl ethyl ketone, acetic acid, methyl alcohol, ethyl alcohol, propyl alcohol, benzyl alcohol, or a mixed solvent thereof.

The type of the biodegradable polymer is not particularly limited, but preferably, polyester may be used, and in particular, the type of the biodegradable polymer may be selected from the group consisting of polylactide, polyglycolide, poly(lactide-co-glycolide), poly(lactide-co-glycolide)glucose, polycaprolactone, and a mixture thereof.

The type of the drug is not particularly limited, and for example, may be selected from dementia drugs; Parkinson's disease drugs; anticancer drugs; antipsychotic drugs such as anti-anxiety drugs, antidepressants, tranquilizers and psychiatric drugs; cardiovascular treatments such as hyperlipidemia drugs, hypertension drugs, hypotension drugs, antithrombotic drugs, vascular relaxants, and arrhythmia drugs; epilepsy treatments; gastrointestinal treatments such as antiulcer drugs; rheumatoid drugs; antispasmodics; tuberculosis drugs; muscle relaxants; osteoporosis treatments; impotence drugs; hemostatic drugs; hormone drugs such as sex hormones; diabetes treatments; antifungal drugs; antifungal drugs; antiviral drugs; antipyretic anti-inflammatory drugs; autonomic neurosuppressants; corticosteroids; diuretics; pain relievers; anesthetics; antihistamines; anti-protozoal drugs; anti-anemia drugs; anti-asthmatic drugs; anticonvulsants; antidotes; antimigraine drugs; anti-emetic drugs; anti-Parkinson drugs; anti-epileptic drugs; anti-platelet drugs; antitussive expectorant; bronchodilators; cardiotonic drugs; immunomodulators; protein drugs; gene drugs; and a mixture thereof.

The types of the above-described drugs are not particularly limited, but may preferably be selected from the group consisting of donepezil, memantine, rivastigmine, entecavir, lamivudine, rotigotine, ropinirole, bupivacaine, ropivacaine, meloxicam, buprenorphine, fentanyl, nimodipine, granisetron, triamcinolone, cytarabine, carmustine, tamsulosin, polmacoxib, testosterone, estradiol, risperidone, paliperidone, olanzapine, aripiprazole, goserelin, leuprolide, triptorelin, buserelin, nafarelin, deslorelin, octreotide, pasireotide, lanreotide, vapreotide, exenatide, liraglutide, lixisenatide, semaglutide, salts thereof, and a mixture thereof.

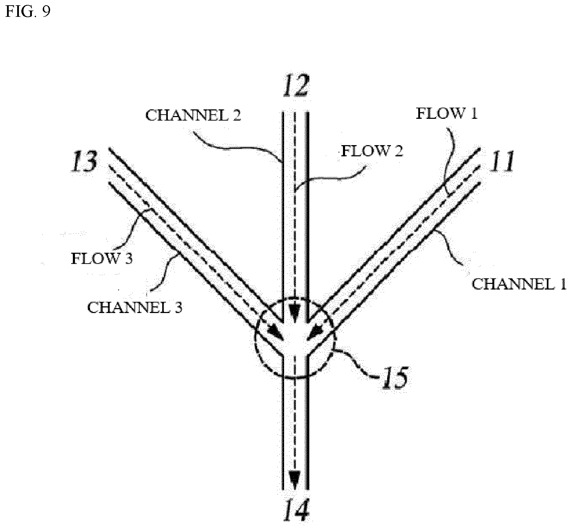

The emulsion generation unit 30 may receive the first raw material and the second raw material from the first raw material storage 10 and the second fuel storage 20 and continuously generate an emulsion including the first raw material in a continuous phase and the second raw material in a dispersed phase using the first raw material and the second raw material. The emulsion generation unit 30 may include an apparatus for preparing a microsphere. The apparatus for preparing a microsphere may be a microchip that forms an emulsion using micro fluidics (a detailed principle of formation of microspheres using a microchip will be described below in FIG. 9).

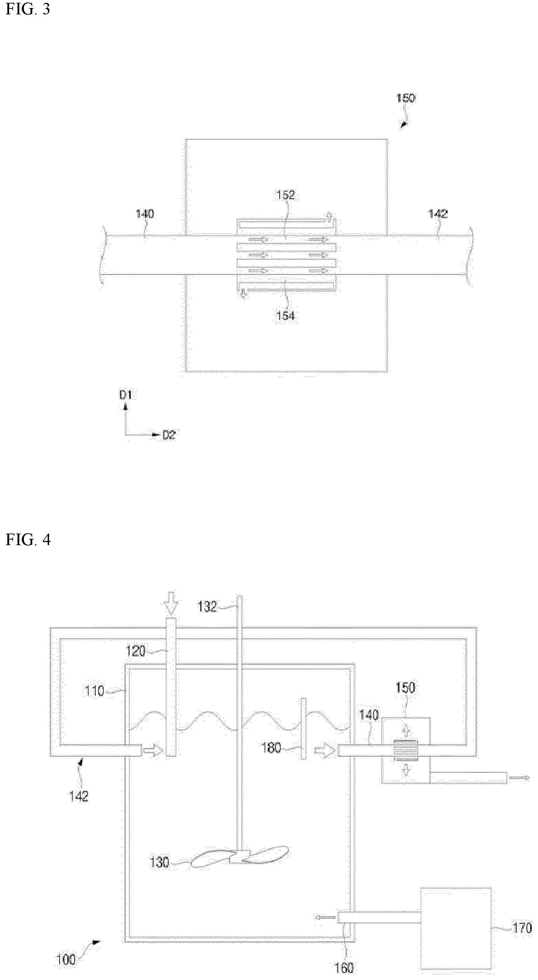

The solvent extraction removal unit 40 receives and accommodates the emulsion generated from the emulsion generation unit 30 and extracts and removes the solvent from the dispersed phase of the emulsion to form microspheres including a drug. The solvent extraction removal unit 40 may include a solvent removing apparatus, and detailed description of the solvent removing apparatus will be described below with reference to FIGS. 2 and 3.

The cleaning unit 50 may recover and clean the microspheres generated from the solvent extraction removal unit 40. A method of recovering and cleaning microspheres from the continuous phase including the microspheres formed from the solvent extraction removal unit 40 is not particularly limited, and the microspheres may be recovered using a method such as filtration or centrifugation, and then cleaned using water. Therefore, the remaining organic solvent and surfactant (e.g., polyvinyl alcohol) may be removed. The cleaning operation may be performed using water, and the cleaning operation may be repeated several times.

The drying unit 60 may dry the cleaned microspheres to obtain microsphere powder. After the filtration and cleaning operations, the obtained microspheres may be dried using typical drying methods to finally obtain dried microsphere powder. The method of drying the microspheres is not limited. However, the drying method used is not particularly limited and may be performed using a freeze drying, vacuum drying, or reduced pressure drying method.

Through the process of drying the microspheres, the intended monodisperse biodegradable polymer-based microsphere powder may be finally prepared, and then the obtained microsphere powder may be suspended in a suspension to fill an appropriate container, such as a disposable syringe, to obtain the final product.

FIG. 2 is a view showing the solvent removing apparatus according to one embodiment of the present disclosure. FIG. 3 is a view showing a filter unit of the solvent removing apparatus of FIG. 2.

Referring to FIGS. 2 and 3, the solvent removing apparatus 100 may include a container 110, an impeller 130, a rotational shaft 132, an emulsion provision unit 120, a filter inlet flow path 140, a filter discharge flow path 142, a filter unit 150, a supply flow path 160, and a supply unit 170. The solvent removing apparatus 100 may further include a filter flow meter 144 and a supply unit flow meter 164.

The container 110 accommodates an emulsion including a first raw material in a continuous phase and a second raw material in a dispersed phase. The container includes a bottom surface and a wall surface that form a space in which the emulsion is accommodated. The container may have a cylindrical shape, but is not limited thereto.

The impeller 130 may be disposed in the container 110 and rotated in the container 110 to stir the emulsion by generating a flow. A rotational flow may be generated in the emulsion by the rotation of the impeller 130, thereby accelerating evaporation of the solvent in the emulsion. The impeller 130 may have a propeller shape having a plurality of blades, but is not limited thereto.

The rotational shaft 132 may be positioned inside the container 110. The impeller 130 may be connected to the rotational shaft 132, and the impeller 130 may be rotated by the rotation of the rotational shaft 132 and may stir the emulsion inside the container 110.

The emulsion provision unit 120 may provide an emulsion including a first raw material in a continuous phase and a second raw material in a dispersed phase inside the container 110. The emulsion may be generated by the emulsion generation unit including a microchip or the like as described in FIG. 1.

The filter unit 150 may be connected to the inside of the container 110 through the filter inlet flow path 140, may receive the emulsion from the container 110 through the filter inlet flow path 140, filter a portion of the continuous phase of the emulsion and the solvent in the continuous phase, and then provide the remaining emulsion back to the container 110.

The filter unit may include a tangential flow filter (TFF) (or cross flow filter). The TFF may filter the solvent extracted from the dispersed phase and present in the continuous phase and a portion of the continuous phase without filtering the dispersed phase in the emulsion. A significant portion of the emulsion provided to the TFF may return to the container 110 through the filter discharge flow path 142.

The TFF includes a filter membrane 154 and a flow path 152, and the flow path is connected to the filter inlet flow path 140 and the filter discharge flow path 142.

The TFF performs filtration by moving a fluid in a direction (D2 direction, the second direction in the drawing) of the flow path 152 parallel to a surface of the filter membrane 154 and filtering the fluid in a direction (D 1 direction, the first direction) perpendicular to the direction of the flow path 152. Therefore, since the clogging phenomenon on the surface of the filter membrane can be prevented and the lifetime of the filter membrane can be extended compared to a normal flow filter (NFF), the TFF is suitable for mass production and is suitable for the solvent removing apparatus of the present disclosure for mass production of microspheres.

The TFF may have the filter membrane 154 to filter particles of 10 µm (micrometers) or less, and thus the solvent and some continuous phases in the emulsion may be filtered through the filter membrane 154, and the remaining continuous phase and dispersed phase may not pass through the filter membrane and may be returned to the container 110.

The filter inlet flow path 140 may be connected to the container 110 at the top of the container 110. Portions of the filter inlet flow path 140 and the filter discharge flow path 142 connected to the container 110 may be formed at substantially the same height. The filter discharge flow path 142 may be connected to the container 110 at a lower position than the filter inlet flow path 140, but in this case, since the emulsion passing through the filter unit 150 through the filter inlet flow path 140 needs to be pressed to move to the container 110 through the filter discharge flow path 142, an additional pressing device is required.

The portions of the filter discharge flow path 142 and the filter inlet flow path 140 connected to the container 110 may preferably be disposed spaced apart from each other at the same height as much as possible.

The supply unit 170 may be connected to the inside of the container 110 and may supply the first raw material to the inside of the container 110. The supply unit 170 may replenish the continuous phase (first raw material) discharged through the filter unit 150 back into the container to maintain the total capacity of the continuous phase. The extraction efficiency of the solvent in the dispersed phase in the emulsion may be related to the amount of the continuous phase and the amount of the solvent in the continuous phase, the solvent in the continuous phase may be removed using the filter unit 150, and a new continuous phase (purified water or a solution including the purified water and a surfactant as the first raw material) may be provided through the supply unit 170 in an amount equivalent to the amount of the continuous phase lost at that time, thereby lowering the concentration of the solvent in the continuous phase and maintaining or increasing solvent extraction efficiency.

The supply flow path 160 may be connected to the container 110 at the bottom of the container 110.

The filter flow meter 144 and the supply unit flow meter 164 may measure a flow rate of the fluid filtered from the filter unit 110 and a flow rate supplied from the supply unit 170 to the container 110, respectively, and control the supply amount from the supply unit 170, thereby maintaining the constant amount of the fluid in the container 110. That is, the first material equivalent to the amount of the first raw material filtered by the filter unit 150 or the amount of the solvent and first raw material filtered by the filter unit 150 may be supplied from the supply unit 170 to the container 110, thereby maintaining the constant fluid level of the emulsion in the container 110.

Although not shown, the solvent removing apparatus may further include a heating device for heating the emulsion in the container 110. By heating the emulsion, the solvent extracted from the dispersed phase may evaporate from the surface, thereby accelerating the extraction and removal of the solvent.

In this case, a temperature of the first raw material supplied from the supply unit 170 may be lower than a temperature of the emulsion provided from the container 110 to the filter unit 170. Since the supply flow path 160 is connected to the bottom of the container 110 and the filter supply flow path 140 is connected to the top of the container 110, as a cold-temperature fluid supplied to the bottom of the container 110 may be heated and stirred inside the container 110, the solvent may be extracted from the dispersed phase, and thus the solution that has the increased concentration of the solvent and is heated may move to the top of the container due to a density difference and return to the inside of the container 110 after the concentration of the solvent decreases through the filter unit 170. Therefore, the solvent can be efficiently removed.

FIG. 4 is a view showing a solvent removing apparatus according to another embodiment of the present disclosure.

Referring to FIG. 4, the solvent removing apparatus 100 may further include a fluid level sensor 180 for measuring a fluid level in the container instead of a flow meter and a component for controlling the amount of the first raw material supplied in order to maintain the fluid level in the container. Since the solvent removing apparatus 100 is substantially the same as the solvent removing apparatus of FIG. 2 except for the fluid level sensor 180, detailed description thereof will be omitted.

The fluid level sensor 180 may measure the fluid level in the container 110 and adjust the amount of the first raw material supplied from the supply unit 160 using the measured fluid level, thereby controlling the constant fluid level in the container 110. The fluid level sensor 180 may include a sensor for measuring a solution level in the container, and various known sensors for measuring a fluid level may be used.

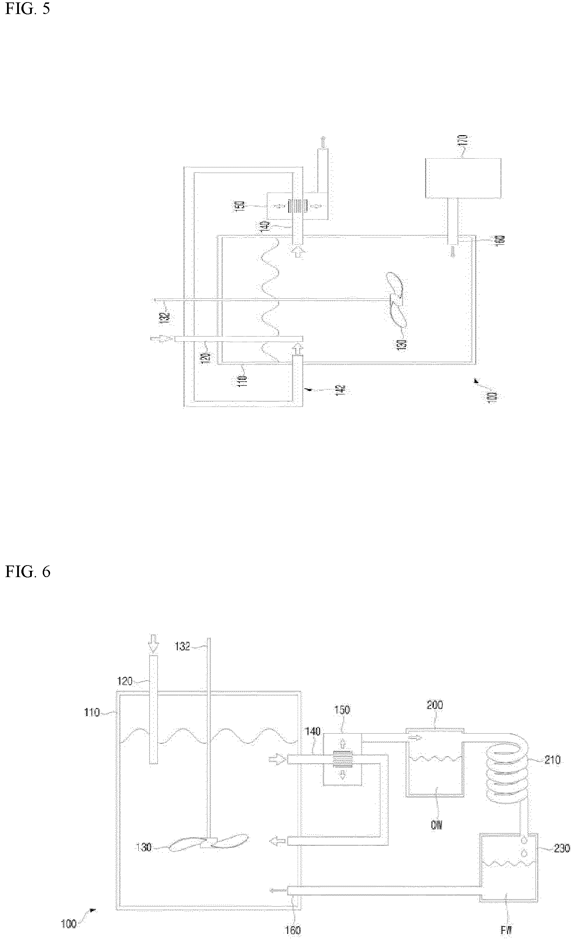

FIG. 5 is a view showing a solvent removing apparatus according to still another embodiment of the present disclosure.

Referring to FIG. 5, the solvent removing apparatus is substantially the same as the solvent removing apparatus of FIG. 4 except for adjusting the fluid level by adjusting a feeding speed of the fluid without the fluid level sensor. Therefore, overlapping description will be omitted.

Since a feeding speed of the emulsion provided from the container 110 to the filter unit 150 is faster than a feeding speed of the first fluid provided from the supply unit 170, the fluid level in the container 110 can be maintained at a portion of the filter unit 150 connected to the inside of the container, that is, a portion of the filter inlet flow path 140 formed. Therefore, an appropriate fluid level can be maintained without a separate fluid level sensor or flow meter.

FIG. 6 is a view showing a solvent removing apparatus according to yet another embodiment of the present disclosure.

Referring to FIG. 6, the solvent removing apparatus is substantially the same as the solvent removing apparatus of FIG. 2 except for further including an evaporation condensation unit. Therefore, overlapping description will be omitted.

The solvent removing apparatus may further include an evaporation condensation unit for evaporating portions of the solvent and first raw material filtered from the filter unit 150 and condensing only the first raw material. The evaporation condensation unit may include an evaporation unit 200, a condensation unit 210, and a supply fluid storage 230.

In the evaporation unit 200, the solvent and first raw material filtered from the filter unit 150 may be heated and evaporated. In this case, since the solvent and the first raw material evaporate and vaporize at different temperatures, only the first raw material may be selectively obtained, cooled through the condensation unit 210, and then stored in the supply fluid storage 230. Therefore, the first raw material accommodated in the supply fluid storage 230 may have a lower temperature than the solution in the container 110. In addition, not only the solvent but also impurity residues in the solution may be removed from the evaporation unit 200, and thus a first raw material OW including the solvent and impurities may be purified into a fresh first raw material FW because the solvent and impurities are removed through the condensation unit 210 and the supply fluid storage 230.

The first raw material recovered from the evaporation and condensation unit may be provided back to the container 110 through the supply unit 170 (see FIG. 2) and the supply flow path 160.

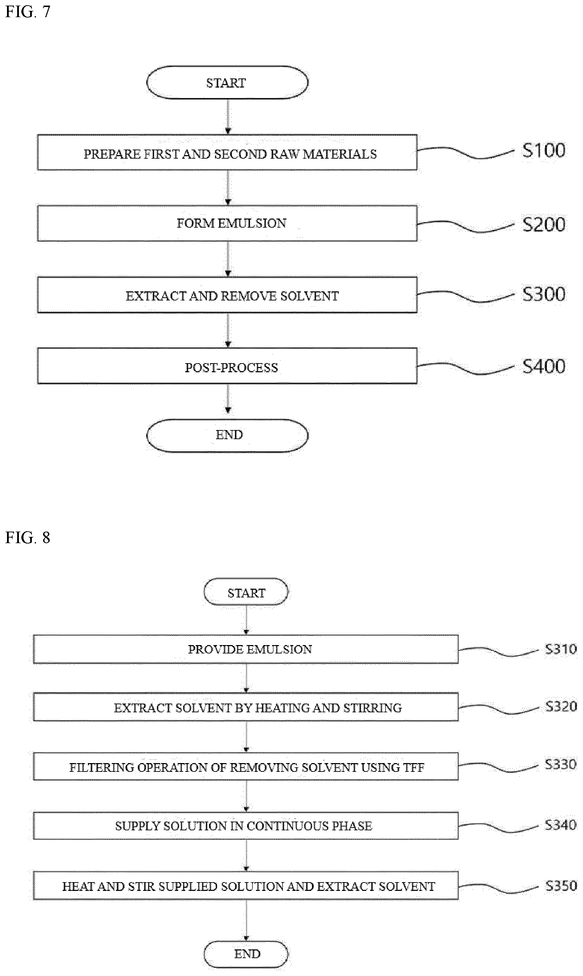

FIG. 7 is a flowchart showing a method of preparing a microsphere according to one embodiment of the present disclosure.

Referring to FIG. 7, the method of preparing a microsphere may include a first and second raw material preparing operation S 100, an emulsion forming operation S200, a solvent extracting and removing operation S300, and a post-processing operation S400.

The first and second raw material preparing operation S100 may include preparing a first raw material and preparing a second raw material including a biodegradable polymer, a drug, and a solvent.

In this case, the first raw material may include purified water and a surfactant. The second raw material may be an oil-phase solution and may include an organic solvent, a biodegradable polymer dissolved therein, and a drug.

The emulsion forming operation S200 may include forming an emulsion including the first raw material in a continuous phase and the second raw material in a dispersed phase using the first raw material and the second raw material. For example, a method of mixing and stirring the first raw material and the second raw material, a microfluidic method of forming microsphere droplets through the flow of microchannels, or the like may be used. The emulsion includes the first raw material in the continuous phase and the second raw material in the dispersed phase.

The solvent extracting and removing operation S300 may include extracting, evaporating, and removing the solvent in the dispersed phase of the emulsion. For example, using the solvent removing apparatus according to embodiments of the present disclosure, the solvent may be extracted from the dispersed phase to the continuous phase of the emulsion, and the extracted solvent may be evaporated and removed. Therefore, solidified microspheres may be formed.

The post-processing operation S400 may include cleaning and drying the solidified microspheres and finally preparing the desired monodisperse biodegradable polymer-based microsphere powder, and thereafter, suspending the obtained microsphere powder in a suspension and filling an appropriate container, for example, a disposable syringe, to perform a post-processing process for obtaining a final product.

FIG. 8 is a flowchart showing a solvent extracting removing operation of the method of preparing a microsphere of FIG. 7.

Referring to FIG. 8, the solvent extracting and removing operation may include an operation S310 of providing an emulsion, an operation S320 of extracting a solvent by heating and stirring, a filtering operation S330 of removing a solvent using a TFF, an operation S340 of supplying a solution in a continuous phase, and an operation S350 of extracting a solvent by heating and stirring the supplied solution.

The solvent extracting and removing operation may be performed using the solvent removing apparatus according to the embodiments of the present disclosure. The solvent removing apparatus may include a container, a filter unit connected to the inside of the container, a supply unit connected to the inside of the container, and a stirring device disposed in the container.

The operation S310 of providing the emulsion may include providing the emulsion to the container. The emulsion includes a first raw material in a continuous phase and a second raw material in a dispersed phase. The first raw material may include purified water and a surfactant, and the second raw material may include an organic solvent, a biodegradable polymer, and a drug.

The operation S320 of extracting the solvent by heating and stirring may include heating the emulsion in the container to accelerate the extraction and evaporation of the solvent in the dispersed phase.

In the filtering operation S330 of removing the solvent using a tangential flow filter (TFF), the filter unit may receive the emulsion at the top of the container and filter a portion of the continuous phase and the solvent in the continuous phase. The filter unit may include the TFF, and the TFF may be formed to filter particles of 10 µm (micrometers) or less.

The operation S340 of supplying the solution in the continuous phase may include providing the solution in the continuous phase (first raw material) to the bottom of the container. In this case, a temperature of the first raw material supplied from the supply unit may be lower than a temperature of the emulsion provided from the container to the filter unit.

In the operation S350 of heating and stirring the supplied solution to extract the solvent, as the cold-temperature solution supplied to the bottom of the container is heated and stirred in the container, the solvent may be extracted from the dispersed phase, and thus the solution that has the increased concentration of the solvent and is heated may move to the top of the container due to a density difference and return to the inside of the container after the concentration of the solvent decreases through the filter unit 170. Therefore, the solvent can be efficiently removed.

According to one embodiment, the solvent extracting and removing operation may further include an evaporating and condensing operation of evaporating the solvent and a portion of the first raw material that are filtered by the filter unit and condensing only the first raw material. The evaporation and condensation of the first raw material may be performed by the evaporation condensation unit further included in the solvent extraction removing apparatus, and the evaporation condensation unit may evaporate the solvent and the portion of the first raw material that are filtered by the filter unit and condense only the first raw material. In this case, in the supplying operation, the first raw material recovered from the evaporation condensation unit may be supplied to the container so that the solvent removing apparatus may entirely form a cycle.

FIG. 9 is a schematic view for describing a detailed principle of formation of microspheres using a microchip.

Referring to FIG. 9, the microchip includes three microchannels (Channel 1, Channel 2, and Channel 3 in the drawing). One of the microchannels is a passage through which a biodegradable polymer solution flows (Flow 2), and the remaining two microchannels are passages through which a water-phase solution flows (Flow 1, Flow 3). The microchannel that accommodates the flow of the biodegradable polymer solution is positioned between the microchannels that accommodate the flow of the water-phase solution. The microchannels that accommodate the flow of the water-phase solution merge with the microchannels that accommodate the flow of the biodegradable polymer-phase solution at a Ψ angle at a merging point 15. Due to interaction of the solutions flowing through the microchannels and immiscibility of the solutions meeting at the merging point, biodegradable polymer-based microsphere droplets are formed at the merging point. The microchannels that accommodate the flow of the water-phase solution each include one of inlets 11 and 13 through which a solution flows into the microchannels. The microchannels that accommodate the flow of the biodegradable polymer-phase solution include an inlet 12 and an outlet 14. The solution containing the biodegradable polymer-based microsphere droplets (dispersed phase) is discharged from the microchip through the outlet 14. The number of microchannels in the microchip for accommodating the flows of solutions may vary depending on required values for a final product.

In the present disclosure, the water-phase solution flows into the microchip through each of the inlets 11 and 13 to form Flow 1 and Flow 3 in the microchannels 1 and 3, respectively, and meets Flow 2 of the biodegradable polymer-phase solution at a predetermined angle at the merging point 15. Here, the microsphere droplets are formed due to the segmentation of the biodegradable polymer solution, that is, Flow 2, by the water-phase solution, that is, Flow 1 and Flow 3, and the immiscibility of the two solutions. Therefore, an emulsion including a dispersed phase and a continuous phase is formed, and the microsphere droplets (dispersed phase) formed at the merging point 15 are discharged out of the microchip through the outlet 14.

According to the embodiments of the present disclosure, a solvent removing apparatus includes a container accommodating an emulsion including a first raw material in a continuous phase and a second raw material in a dispersed phase, a filter unit connected to the inside of the container, receiving the emulsion from the container, filtering a portion of the continuous phase of the emulsion and the solvent in the continuous phase, and then supplying the remaining emulsion back to the container, a supply unit connected to the inside of the container and supplying the first raw material to the inside the container, and a stirring device for stirring the emulsion in the container by generating a flow. Since the solvent in the dispersed phase of the emulsion is extracted and evaporated in the continuous phase, the solvent extracted in the continuous phase is removed by the filter unit, and the first raw material is supplied and circulated by the supply unit, it is possible to efficiently extract and remove the solvent.

Although the present disclosure has been described above with reference to the embodiments, those skilled in the art will understand that the present disclosure may be modified and changed in various ways without departing from the spirit and scope of the present disclosure as described in the appended claims.

### [DESCRIPTION OF DRAWING SYMBOLS]

| | | | |
|---|---|---|---|
| 10: | first raw material storage | 20: | second raw material storage |
| 30: | emulsion generation unit | 40: | solvent extraction removal unit |
| 50: | cleaning unit | 60: | drying unit |
| 100: | solvent removing apparatus | 110: | container |
| 120: | emulsion provision unit | 130: | impeller |
| 170: | supply unit | | |

## Claims

1. A solvent removing apparatus comprising:
a container that accommodates an emulsion including a first raw material in a continuous phase and a second raw material in a dispersed phase;
a filter unit connected to the inside of the container to receive the emulsion from the container and filter a solvent in the continuous phase together with a portion of the continuous phase of the emulsion;
a supply unit connected to the inside of the container to supply the first raw material to the inside of the container; and
a stirring device that stirs the emulsion in the container by generating a flow,

2. The solvent removing apparatus of claim 1, wherein the filter unit includes a tangential flow filter (TFF) formed to filter the portion of the continuous phase of the emulsion and the solvent in the continuous phase and then provide the remaining emulsion back to the container, and the TFF filters the solvent and a portion of the first raw material without filtering the dispersed phase of the emulsion.

3. The solvent removing apparatus of claim 2, wherein the TFF filters particles of 10 µm (micrometers) or less.

4. The solvent removing apparatus of claim 1, further comprising an evaporation condensation unit that evaporates the filtered solvent and portion of the first material and condenses only the first raw material,
wherein the first raw material recovered from the evaporation condensation unit is provided to the supply unit to supply the recovered first raw material back to the container.

5. The solvent removing apparatus of claim 1, wherein a first raw material equivalent to the amount of the first raw material filtered by the filter unit or the amount of the solvent and first raw material filtered by the filter unit is supplied from the supply unit to the container to maintain a constant fluid level in the container.

6. The solvent removing apparatus of claim 1, further comprising a fluid level sensor that measures a fluid level in the container,
wherein the fluid level in the container is constantly controlled by adjusting the amount of the first raw material supplied from the supply unit using the measured fluid level.

7. The solvent removing apparatus of claim 1, wherein a portion of the filter unit connected to the container is formed at the top of the container, and
the supply unit provides the first raw material to the bottom of the container.

8. The solvent removing apparatus of claim 7, further comprising a heating device that heats the emulsion in the container.

9. The solvent removing apparatus of claim 7, wherein a feeding speed of the emulsion provided from the container to the filter unit is faster than a feeding speed of the first raw material provided from the supply unit so that the fluid level in the container is maintained at the portion of the filter unit connected to the inside of the container.

10. The solvent removing apparatus of claim 1, wherein a temperature of the first raw material supplied from the supply unit is lower than a temperature of the emulsion provided from the container to the filter unit.

11. The solvent removing apparatus of claim 1, wherein the first raw material of the emulsion includes purified water and a surfactant, and the second raw material may include an organic solvent, a biodegradable polymer, and a drug.

12. A method of preparing a microsphere using a solvent removing apparatus including a container, a filter unit connected to an inside of the container, a supply unit connected to the inside of the container, and a stirring device disposed in the container, the method comprising:
an operation of preparing a first raw material and a second raw material including a biodegradable polymer, a drug, and a solvent;
an operation of forming an emulsion including the first raw material in a continuous phase and the second raw material in a dispersed phase using the first raw material and the second raw material;
an operation of providing the emulsion to the inside of the container of the solvent removing apparatus;
an operation of stirring the emulsion using the stirring device in the solvent removing apparatus and extracting the solvent in the dispersed phase of the emulsion as the continuous phase;
an operation of filtering a portion of the continuous phase including the extracted solvent and the solvent in the continuous phase using the filter unit and then providing the remaining emulsion back to the container; and
an operation of supplying a solution in a continuous phase to the container.

13. The method of claim 12, wherein in the operation of the filtering, the filter unit includes a tangential flow filter (TFF), and the TFF filters particles of 10 µm (micrometers) or less in order to filter a solvent and a portion of the first raw material without filtering the dispersed phase of the emulsion.

14. The method of claim 12, wherein the extracting operation includes heating the emulsion in the container to accelerate extraction and evaporation of the solvent in the dispersed phase, and
in the supplying operation, a temperature of the first raw material supplied from the supply unit is lower than a temperature of the emulsion provided from the container to the filter unit.

15. The method of claim 12, further comprising an evaporating and condensing operation of evaporating the solvent and portion of the first material filtered by the filter unit and condensing only the first raw material,
wherein the supplying operation includes supplying a first raw material recovered from the evaporation condensation unit to the container.

16. The method of claim 12, wherein in the operation of the filtering, the filter unit receives the emulsion at the top of the container and filters a portion of the continuous phase and the solvent in the continuous phase, and
in the supplying operation, the solution in the continuous phase is provided to the bottom of the container.

17. The method of claim 12, wherein in the preparing operation, the first raw material includes purified water and a surfactant, and the second raw material includes an organic solvent, a biodegradable polymer, and a drug.
